Europäisches Patentamt

European Patent Office

Office européen des brevets

(11) Numéro de publication: **0 318 543 B1**

(12) **FASCICULE DE BREVET EUROPEEN**

(45) Date de publication de fascicule du brevet: **29.06.94** (51) Int. Cl.⁵: **C12P 19/04**

(21) Numéro de dépôt: **88904978.9**

(22) Date de dépôt: **26.05.88**

(86) Numéro de dépôt internationale :
**PCT/FR88/00266**

(87) Numéro de publication internationale :
**WO 88/09381 (01.12.88 88/26)**

(54) **PROCEDE DE PRODUCTION DE CELLULOSE BACTERIENNE A PARTIR DE MATIERE D'ORIGINE VEGETALE.**

(30) Priorité: **26.05.87 FR 8707387**

(43) Date de publication de la demande:
**07.06.89 Bulletin 89/23**

(45) Mention de la délivrance du brevet:
**29.06.94 Bulletin 94/26**

(84) Etats contractants désignés:
**AT BE CH DE FR GB IT LI LU NL SE**

(56) Documents cités:
**EP-A- 0 228 779**
**AU-A-64 160 /86**
**GB-A- 1 570 487**

**Cellulose Chemistry and Technology, vol. 59, no. 5, septembre/octobre 1975, E. Correns et al.; pp. 449-469**

(73) Titulaire: **UNION FINANCIERE POUR LE DEVE-LOPPEMENT DE L'ECONOMIE CEREALIERE-UNIGRAINS**
**8, avenue du Président Wilson**
**F-75116 Paris(FR)**

Titulaire: **LABOUREUR, Pierre François**
**2, rue Victor-Daix**
**F-92200 Neuilly-sur-Seine(FR)**

(72) Inventeur: **LABOUREUR, Pierre Francois**
**2, rue Victor-Daix**
**F-92200 Neuilly-sur-Seine(FR)**

(74) Mandataire: **Ahner, Francis et al**
**CABINET REGIMBEAU**
**26, avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

La présente invention concerne un procédé de production de cellulose par voie microbiologique.

Les végétaux supérieurs constituent la matière première à partir de laquelle la cellulose industrielle est obtenue; elle en est l'un des constituants majeurs, puisqu'elle représente, en général, 30 à 50% du poids de ces végétaux.

Les plus utilisés, industriellement, sont les arbres: conifères et feuillus et, dans une proportion plus faible, les céréales, dont les pailles peuvent également servir de source de cellulose.

La durée de renouvellement de ces matières premières est de plusieurs dizaines d'années pour les arbres et de une année pour les céréales.

Par ailleurs, les "gisements" importants de matières cellulosiques effectivement exploitables, pour leur transformation en cellulose, sont très inégalement répartis dans le monde.

Chez les végétaux supérieurs, la cellulose est souvent, du point de vue pondéral, le constituant le plus important, mais elle est accompagnée, en quantités variables, de lignines, d'hemicelluloses, de composés pectiques et de nombreuses autres substances qui sont associées, ou combinées ("incrustées") à la cellulose.

Ces substances non cellulosiques, qui représentent 50 à 70% de la matière végétale, doivent être séparées, et éliminées, au moins partiellement pour obtenir de la cellulose purifiée, ou semipurifiée, industriellement utilisable.

Le prix de revient de la cellulose obtenue selon les procédés conventionnels, est dû, dans une proportion de 60% à 80% au coût: de l'énergie, des produits chimiques et des opérations industrielles, nécessaires à la séparation et à l'élimination des substances non cellulosiques, ainsi qu'à la purification, (dont le blanchiment) de la cellulose elle-même, en particulier pour ce qui concerne les qualités purifiées et semi-purifiées de cellulose.

La présente invention due aux travaux de Monsieur P. LABOUREUR a pour objet un procédé industriel de fabrication de cellulose bactérienne pour tous usages, principalement chimiques, mais également papetiers.

- Avec le procédé selon l'invention, il est possible:
  (1) d'obtenir, par biosynthèse et/ou bioconversion microbienne, de la cellulose, dans des temps très courts: 1 à 4 jours (au lieu de 1 année à plusieurs dizaines d'années),
  (2) d'obtenir directement de la cellulose purifiée ou semi-purifiée, sans avoir à mettre en oeuvre des techniques complexes et onéreuses pour séparer et éliminer les constituants non cellulosiques, comme dans les procédés conventionnels,
  (3) de supprimer, pour l'essentiel, les problèmes d'élimination des déchets et sous-produits non-cellulosiques,
  (4) d'utiliser, pour cette production comme matières premières principales, des produits d'origine agricole, économiques, largement disponibles, et rapidement renouvelables.

Plus particulièrement, la présente invention concerne un procédé de production de cellulose d'origine bactérienne à partir de matières d'origine végétale contenant des hydrates de carbone, dans un bioréacteur en culture submergée, caractérisé en ce que dans le milieu de production comprenant lesdits hydrates de carbone, on cultive au moins une souche bactérienne productrice de cellulose, en ce que ledit bioréacteur comporte des moyens permettant la fixation des microorganismes et le développement des fibres de cellulose et en ce que pendant la phase de production de la cellulose, l'aération est faible et l'agitation est maintenue avec un cisaillement minimum afin de favoriser le développement des fibres de cellulose.

La souche bactérienne utilisée comme agent de biosynthèse et/ou de bioconversion est de préférence:
(1) une bactérie susceptible de produire de la cellulose, en utilisant comme matière principale des hydrates de carbone, économiques, et largement disponibles,
(2) une bactérie dont la croissance est suffisamment rapide et qui produit la cellulose dans un temps inférieur à 96 heures et généralement inférieur à 48 heures,
(3) une bactérie, capable de produire de la cellulose en culture submergée, à grande échelle, c'est-à-dire dans un bioréacteur de type industriel,
(4) une bactérie dont le rendement et la productivité sont élevés,
(5) une bactérie produisant une cellulose purifiée, dont les caractéristiques sont compatibles avec les utilisations industrielles,
(6) une bactérie dont la production de cellulose est essentiellement exocellulaire,
(7) une bactérie pouvant se développer dans des conditions sélectives.

En effet, la biosynthèse de la cellulose, par divers types de microorganismes et plus particulièrement de bactéries, est connue depuis longtemps, mais cette cellulose n'a été obtenue qu'en culture statique, ou

2

EP 0 318 543 B1

en culture agitée à l'échelle laboratoire, pas en bioréacteurs industriels (de grande dimension) en culture submergée, ce qui exclut toute véritable production industrielle, et dans des temps généralement longs: une ou plusieurs semaines ce qui est également incompatible avec une production industrielle.

L'utilisation répondant aux caractéristiques ci-dessus, comme agents de biosynthèse et/ou de biconversion, de souches bactériennes sélectionnées permet la mise en oeuvre d'un procédé de production industrielle de cellulose bactérienne.

Parmi les microorganismes étudiés pour la biosynthèse de cellulose, on peut citer des algues unicellulaires à croissance rapide, par exemple, du genre Oocystis ou Glaucocystis, des champignons inférieurs, en particulier des oomycetes, par exemple des genres: Pythium, Phytophtora, Saprolegnia, Achyla, mais pour diverses raisons, ces microorganismes semblent difficilement utilisables à l'échelle industrielle (i).

Par contre, en raison de leurs caractéristiques, certaines bactéries peuvent être envisagées comme agents de biosynthèse, et/ou de bioconversion, à l'échelle industrielle, en particulier, mais non

(i) vitesse de croissance et/ou de production insuffisantes; rendements et/ou productivité insuffisants: exigences nutritionnelles; difficultés technologiques, etc.

exclusivement des souches sélectionnées appartenant aux genres et espèces ci-dessous:

Achromobacter
Agrobacterium
Aérobacter
Alcaligènes
Azotobacter
Gluconobacter
Rhizobium
Sarcina
Pseudomonas
Brevibacterium
Flavobacterium
Erwinia,
mais plus particulièrement:
Acétotacter aceti
Acetobacter aceti subsp-xylinum
Acetobacter acetigenum
Acetobacter pastorianus
Acetobacter hansenii
Acetobacter liquefaciens
Acetobacter sp

La production de cellulose, dans des conditions industrielles est la propriété de certaines souches sélectionnées, mais pas de toutes les souches d'un même genre, d'une même espèce ou sous espèce.

Toutes les souches d'Acetobacter, même celles réputées produire de la cellulose en cultures statiques, ou agitées à l'échelle laboratoire ne produisent pas nécessairement de la cellulose en quantité significative, en bioréacteur, à l'échelle et dans les conditions industrielles.

Le procédé selon la présente invention vise à permettre une production à l'échelle industrielle.

Pour ce faire, le procédé selon l'invention de production industrielle de cellulose bactérienne est effectuée en culture submergée, en bioréacteur.

La production peut être obtenue:

- soit en une seule étape (ou opération) c'est-à-dire avec simultanément: développement du microorganisme, agent de biosynthèse et/ou de biconversion, et production de cellulose,
- soit en deux étapes (ou opérations) avec développement du microorganisme agent de biosynthèse et/ou de biconversion, dans une première étape, puis séparation de ce microorganisme, et dans une deuxième étape production de cellulose avec microorganisme non proliférant.

Cette deuxième technique s'apparente aux bioconversions.

Le choix de la technique, en une ou deux étapes, dépend des caractéristiques de la souche utilisée, mais aussi de l'importance quantitative du réactif microbien; si celle-ci est faible, il est généralement plus simple d'opérer en une étape.

Avec la technique en une seule étape, on peut utiliser:

- soit un système discontinu ou batch
- soit un système semi continu ou fed-batch
- soit un système continu.

Cependant, avec le procédé selon l'invention il est recommandé d'utiliser un système discontinu, ou mieux semi-continu, en raison des difficultés liées à la séparation, en continu, à partir du bioréacteur, de la cellulose produite, et du réactif microbien, ainsi que des inconvénients et risques correspondant aux systèmes continus, en général.

Un procédé qui utiliserait, non pas le réactif microbien dans sa totalité, mais une fraction subcellulaire contenant les systèmes enzymatiques et les cofacteurs nécessaires à la transformation du substrat carboné en cellulose peut également être envisagé.

Le procédé selon l'invention, repose sur la mise en évidence des caractéristiques nécessaires à une culture submergée. Comme cela a été indiqué, l'aération doit rester faible durant la phase de production, par exemple inférieure à 1,5 ppm de préférence inférieure à 0,5 ppm d'$O_2$ dissous dans le réacteur.

En outre, l'agitation doit être faible, ce paramètre est difficile à définir car il dépend des caractéristiques du réacteur, et de celles du système d'agitation et également des paramètres du milieu. L'agitation optimum pourra néanmoins être déterminée par l'homme du métier. Il est important que le système d'agitation soit réglable en cours de culture, et puisse être tel que, pendant la phase de production de la cellulose, il n'y ait pas d'effet de turbulence, et ou, de cisaillement qui empêcheraient la formation de la cellulose, puis sa polymérisation, et sa cristallisation et l'organisation des fibrilles; toute agitation excessive a des effets négatifs; en fin de fermentation un simple brassage lent est préférable.

Il est également nécessaire de prévoir des moyens permettant d'assurer la fixation des microorganismes et le développement des fibres de la cellulose. Il peut s'agir de supports de formes diverses ou d'autres dispositifs appropriés au développement des fibres de celluloses, de proche en proche, à partir des sites de fixation. En effet, on a pu mettre en évidence que la cellulose ne se forme que très difficilement dans la phase liquide libre, sans sites de fixation.

Les moyens de fixation peuvent être aussi bien fixes sur le réacteur par exemple des contrepales qu'amovibles tels quec des éléments de garnissage par exemple.

Les milieux de production doivent contenir les éléments nécessaires à la croissance et à l'activité du microorganisme, et à la production optimale de la cellulose.

Lors de la phase de production de cellulose, il n'est pas nécessaire d'avoir une prolifération bactérienne (importante) En effet, celle-ci pourrait se produire au détriment de la cellulose.

A titre d'exemple pour les souches d'Acetobacter selon l'invention, la composition du milieu de production pourra être la suivante:

| | |
|---|---|
| substrat carboné (voir ci-après) | 20 g/l à 150 g/l de préfér. 30 g/l à 60 g/l |
| acide citrique | 0,2 g/l à 6 g/l, de préférence 0,5 g/l à 3 g/l |
| extrait de levure | 0,1 g/l à 2 g/l de préférence 0,5 g/l à 1 g/l |
| ou corn steep | de préférence 0,5 g/l à 1 g/l |
| NH4Cl | 2 g/l à 10 g/l de préférence 3 g/l à 6 g/l |
| ou (NH4)2 SO4ou | de préférence 3 g/l à 6 g/l |
| NH$_4$ NO$_3$ ou urée | de préférence 3 g/l à 6 g/l |
| HK2 PO4 | 0,2 g/l à 0,6 g/l de préfér. 0,3 g/l à 0,5 g/l |
| H2K PO4 | de préfér. 0,3 g/l à 0,5 g/l |
| Mg SO4, 7H20 | 0,05 g/l à 0,5 g/l de préfér. 0,1 g/l à 0,2 g/l |
| HNaCO3 | 0,2 g/l à 0,8 g/l de préfér. 0,3 g/l à 0,6 g/l |
| CaCl2 | 0,5 g/l à 2 g/l de préférence 0,7 g/l à 5 g/l |
| pH | 4,5 à 5,5 de préférence de 4,7 à 4,9 |
| stérilisation | 10' à 20' à 110' à 120' de préférence 15' à 115' |

Le milieu sera de préférence maintenu à un pH en-dessous de pH5, la stérilisation pouvant ainsi être supprimée.

Remarques sur les milieux de production

La nature du substrat carboné, matière première principale, dépend des caractéristiques biochimiques de la souche microbienne utilisée en particulier de son équipement enzymatique, qui lui permet ou non, d'utiliser le substrat carboné.

Pour des raisons scientifiques, techniques et économiques, les substrats carbonés recommandés, en particulier avec les Acetobacter sont:

des hydrates de carbone d'origine végétale qui sont constitués ou qui contiennent des hexoses plus particulièrement du glucose et du fructose ou éventuellement d'autres hexoses ou des pentoses:

(a) le saccharose (avec souches invertase +), sous ses diverses formes: jus de betterave ou de canne, mélasses, sirops, sucre brun, sucre blanc, mélanges glucose- fructose, par exemple; dans certains cas, il peut être utile d'hydrolyser les matières premières contenant du saccharose pour les souches invertase (-) et/ou pour rendre utilisables d'autres hydrates de carbone;

(b) les amidons (avec souches amylases +) et dérivés: farines, amylodextrines- dextrines, maltose, glucose, par exemple,

Dans certains cas, il peut être utile d'effectuer une hydrolyse enzymatique des amidons avec les souches amylases (-) en particulier.

Avec certaines souches, il peut y avoir intérêt à utiliser, ou à ajouter en quantité limitée: éthanol, ou glycérol, ou lactate ou acétate ou manitol qui peuvent stimuler la production de cellulose, ainsi éventuellement que du méthanol.

La concentration en substrat carboné, hydrate de carbone, dans le milieu de production, type saccharose ou amidon, a une très grande importance, cette concentration doit être comprise entre 20 et 150 g/l.

Avec certains microorganismes, en particulier, certains Acetobacter, la production de cellulose est optimale entre 35 et 65 g/l elle est faible à partir de 25 g/l, nulle à plus faible concentration.

La concentration des hydrates de carbone doit être maintenue de préférence entre 30 g/l et 50 g/l pour les Acetobacter au moins pendant la phase de production de la cellulose.

D'autres éléments peuvent avoir une influence sur la production de cellulose. Ainsi, l'acide citrique a un rôle bénéfique avec certains microorganismes, par exemple certaines souches d'Acétobacter.

Quant au magnésium, il est très important pour la production de cellulose par exemple avec certaines souches d'Autobacter.

Le calcium et le fer sont favorables pour certaines souches.

Les autres éléments minéraux et oligo-éléments sont en général apportés en quantité suffisante par les impuretés.

Les besoins en vitamines et facteur de croissance en particulier P.a.b, B1, B2, B6, B12, ac. folique et éventuellement en ac. aminés (ac. glutamique, lysine, etc.) sont couverts par:

-   les extraits de levure,
-   ou le corn steep
-   ou les peptones
-   ou l'inoculum lui-même, en quantité suffisante.

Ces besoins sont spécifiques des souches utilisées.

La conduite de la fermentation doit tenir compte des caractéristiques précédentes.

L'ensemencement du bioréacteur de production est effectué, avec une préculture âgée de 3 à 12 jours, de préférence de 5 à 8 jours. Cette préculture, peut, selon le volume du bioréacteur de production être précédée d'une ou plusieurs autres précultures de volumes croissants.

La ou les préculture(s) sont effectuées à partir d'une culture statique sur milieu d'entretien (en tubes).

La cuture d'entretien, la ou les préculture(s) sont réalisées, en milieu liquide, de même composition, au même pH et à la même température, que le milieu de production.

Les cultures d'entretien doivent être repiquées tous les 20 à 30 jours de préférence.

Les cultures "stock" peuvent être lyophilisées.

L'ensemencement du bioréacteur de production est effectué, avec 5 à 20% en volume de préculture, de préférence avec 10%.

Les conditions de cultures, ainsi que les milieux de cultures dépendent des souches utilisées.

Avec les Acetobacter, la température est maintenue entre 25° et 35°, de préférence entre 28° et 32°, si possible entre 29° et 31°, avec d'autres bactéries, la température peut être différente.

Avec les Acetobacter, le pH de la culture est maintenu entre 4,5 et 5,5, de préférence entre 4,6 et 4,8. Avec d'autres bactéries, le pH de la culture peut être différent.

L'agitation exprimée en tours/minute, variable selon le système d'agitation, doit être contrôlée et limitée, par exemple entre 60 et 90 tours/minute, pendant la phase de développement des bactéries et par exemple entre 30 et 60 tours/minute pendant la phase de production de la cellulose.

Les conditions optimales de l'agitation dépendent de la souche utilisée et des caractéristiques du bioréacteur et de celles du dispositif d'agitation-aération.

L'aération (exprimée en volume d'air, par volume de milieu, par minute: VVm) variable selon le système d'aération, doit être contrôlée et limitée, par exemple égale ou inférieure à 1 VVm pendant la phase de développement des bactéries, et par exemple égale ou inférieure à 0,5 VVm pendant la phase de production de la cellulose.

Les caractéristiques optimales de l'agitation dépendent de la souche utilisée.

D'une façon générale, il est essentiel que les conditions et la conduite de la fermentation, en particulier en ce qui concerne l'agitation et l'aération, soient telles que le développement des bactéries reste très limité, et que le rapport biomasse synthétisé (bactéries)/cellulose produite, soit aussi faible que possible:
- pour augmenter le rapport cellulose produite/substrat carboné utilisé,
- pour limiter et simplifier la séparation: cellulose-biomasse et l'élimination de celle-ci.

Au cours de la culture, les teneurs en substrat carboné, en azote et phosphore sont contrôlées régulièrement.

En particulier les teneurs en azote et en phosphore peuvent être utilisées pour contrôler et limiter la croissance de la biomasse par exemple lors de l'étape de production de cellulose.

Au cours de la culture, le milieu peut être complété en substrat carboné à 1 ou plusieurs reprises, en particulier lorsqu'on atteint la concentration minimum critique en dessous de laquelle il n'y a plus synthèse de la cellulose.

Il peut y avoir intérêt, pour accélérer le développement, à utiliser une concentration initiale faible en substrat carboné, par exemple 10 à 20 g/l, pour l'augmenter ensuite, pendant la phase de production de la cellulose.

En fin de culture, le milieu peut être recyclé après séparation de la cellulose et de la biomasse.

En culture discontinue, ou semi-continue, la durée moyenne de culture est généralement comprise entre 40 et 120 h, de préférence entre 45 et 60 heures. Toutefois, cette durée peut être diminuée en utilisant un inoculum plus important. D'une façon générale, le degré de polymérisation (D.P.) de la cellulose, et son degré de cristallinité et d'organisation:
- augmente avec l'âge de la culture,
- est inversement proportionnel à l'agitation et à l'aération.

En fin de culture, il peut y avoir intérêt à faire suivre la phase de synthèse de la cellulose, d'une phase de "maturation" pendant laquelle l'agitation et l'aération sont réduites au minimum, en évitant l'anaérobiose, pour augmenter les degrés de polymérisation, de cristallinité de la cellulose et l'organisation des fibrilles.

Enfin, le procédé selon l'invention permet une purification simple de la cellulose obtenue.

En effet, le procédé repose en grande partie sur l'obtention d'une biomasse, la plus faible possible, compatible avec une bonne production de cellulose.

La séparation en fin de culture est effectuée par des procédés connus:
- en fin de culture, le mélange cellulose et biomasse est séparé par soutirage, et, ou filtration de la phase liquide;
- le mélange est essoré,
- le mélange est lavé abondamment, à plusieurs reprises avec de l'eau, pour entraîner, physiquement, le maximum de biomasse (bactéries) et d'impuretés (produits solubles et insolubles du milieu de culture).

Il est également possible de soutirer la phase liquide et d'effectuer les opérations de lavage et de purification de la cellulose dans le bioréacteur lui-même.

Après les lavages à l'eau, la cellulose est débarrassée de la plus grande partie des corps microbiens et des impuretés, mais une certaine proportion de corps microbiens restent fixes, ou inclus sur ou dans la cellulose (en fait, sur, ou entre les fibres de cellulose).

La cellulose, lavée à l'eau, est purifiée, par tout traitement physique, physico-chimique, ou chimique permettant de désorber et/ou de lyser (ou solubiliser), puis d'éliminer et d'entraîner, les corps microbiens et éventuellement d'autres impuretés, qui se trouvent encore sur ou entre les fibres de cellulose, après le lavage à l'eau.

En particulier, la purification de la cellulose obtenue est effectuée de préférence par traitement avec un acide organique ou minéral, de préférence l'acide acétique à chaud. Il est possible de combiner ce traitement avec l'utilisation d'agents tensioactifs.

De préférence, la cellulose, après essorage et lavage à l'eau, est traitée à froid ou de préférence à chaud, à 100°, pendant 0,5 à 2 heures de préférence pendant une heure par de l'acide acétique, à raison de 1 à 15% de préférence 5 à 8% en volume par rapport au volume de cellulose hydratée (après essorage).

Après ce traitement à l'acide acétique, la cellulose est lavée abondamment à l'eau.

Si cela est nécessaire, après le lavage à l'eau, la cellulose est neutralisée par une solution diluée de soude, puis de nouveau rincée à l'eau.

Avec certaines matières premières, il peut être nécessaire pour éliminer certaines impuretés ou colorants de compléter le traitement précédent par un traitement à la soude 1 à 5 N. Après ce traitement à la soude, laver abondamment à l'eau, essorer et sécher si nécessaire.

L'élimination des corps microbiens par un acide tel que l'acide acétique est due pour l'essentiel à une action: lyse et/ou désorption (ou "défixation"). Le traitement par l'acide acétique, pour l'élimination des corps microbiens et des impuretés peut éventuellement être remplacé par un traitement avec d'autres acides organiques ou minéraux, combiné ou non avec des produits tensioactifs appropriés.

La cellulose bactérienne purifiée est obtenue avec ce procédé et selon les souches et les conditions de culture, avec des rendements qui peuvent être compris entre 30% et 70%.

$$\text{Rendement: } \frac{\text{poids de cellulose synthétisée}}{\text{poids desubstrat carboné utilisé}}$$

et avec une productivité comprise entre 10 et 100 g/l/J.

La cellulose bactérienne purifiée est de l'α-cellulose ayant un D.P. compris entre 500 et 3500 généralement entre 1000 et 1500; la cellulose bactérienne ainsi obtenue ne contient ni autres polysaccharides, ni hemicelluloses, ni composés pectiques, ni lignines.

La cellulose bactérienne purifiée ainsi obtenue, selon l'invention peut être utilisée comme cellulose chimique pour la fabrication de dérivés de la cellulose, comme l'acétate de cellulose, la nitrocellulose, la carboxyméthylcellulose, l'hydroxyéthylcellulose, la méthylcellulose, l'hydroxyméthylcellulose, etc. et pour des usages papetiers, en particulier en mélange dans les pâtes ou pour d'autres usages.

D'autres caractéristiques de l'invention apparaitront dans les exemples ci-après:

Exemple 1

| - souche - Acetobacter aceti subsp.xylinum ATCC 21780 | |
|---|---|
| - préculture | 160 h, en milieu de production |
| - ensemencement | 12% en volume |
| - bioréacteur | 300 l rempli à 220 l |
| - température | 30° ± 1° |
| - agitation | 60 t-m |
| - aération | 0,6 VVm |
| - stérilisation | 15' à 115' |
| - durée de culture | 45 h |

| - milieu de culture | |
|---|---|
| - saccharose | 50 g/l |
| - ac. citrique | 2 g/l |
| - extrait levure | 0,5 g/l |
| - NH4Cl | 4 g/l |
| - Mg SO4, 8H$_2$0 | 0,1 g/l |
| - HK2PO4 | 0,3 g/l |
| - H2KPO4 | 0,3 g/l |
| - HNaCO3 | 0,5 g/l |
| pH | 4,8 |
| - eau | q.s.p. pour 1 l |

Résultats

| rendement | 18 g/l - saccharose non utilisé, recyclable: 20 g<br>- soit rendement: 60% |
|---|---|
| productivité | 10 g/l/J |
| cellulose purifiée par traitement: | acide acétique<br>- 6% en volume à 100° pendant 30 mn<br>- cellulose -D.P.-1500 |

Exemple 2

| - souche - Acetobacter sp ATCC 8303 | |
|---|---|
| - préculture | 150 h en milieu de production |
| - ensemencement | 8% en volume |
| - bioréacteur | 500 l rempli à 400 l |
| - température | 28° ± 1 |
| - agitation | 80 t/m puis 50 t/m après 36 h |
| - aération | 1 VVm puis 0,5 VVm après 36 h pas de stérilisation |
| - durée de culture | 48 h |

| - milieu de culture | |
|---|---|
| - mélange glucose 80% | 35 g/l |

| maltose 20% | |
|---|---|
| - ac. citrique | 1 g/l |
| - corn steep | 0,3 g/l |
| - extrait levure | 0,3 g/l |
| - (NH4)2 SO4 | 4 g/l |
| - Mg SO4 7H2O | 0,1 g/l |
| - Ca Cl2 | 1 g/l |
| - HK2 PO4 | 0,4 g/l |
| - H2 K PO4 | 0,4 g/l |
| - HNa CO3 | 0,5 g/l |
| - pH | 4,6 |

après 30 h de culture, le milieu est complété avec 20 g/l mélange glucose-maltose.

8

Résultats

| rendement | 27 g/l - mélange non utilisé (glucose + maltose) recyclable: 18 g - soit rendement: 65% |
|---|---|
| productivité cellulose: | 13,5 g/l/J purifiée par traitement à l'acide acétique 1 h à 100°C; 5% en volume, neutralisée à la soude, et lavée, cellulose D.P.-1200 |

**Revendications**

**1.** Procédé de production de cellulose d'origine bactérienne à partir de matière d'origine végétale contenant des hydrates de carbone, dans un bioréacteur en culture submergée, caractérisé en ce que dans le milieu de production comprenant lesdits hydrates de carbone, on cultive au moins une souche bactérienne productrice de cellulose et en ce que ledit bioréacteur comporte dos moyens permettant la fixation des microorganismes et le développement des fibres de cellulose et en ce que pendant la phase de production de la cellulose, l'aération est faible et l'agitation est maintenue avec un cisaillement minimum pour favoriser la croissance des fibres de cellulose.

**2.** Procédé suivant la revendication 1, caractérisé en ce que la souche bactérienne productrice de cellulose appartient aux genres suivants: Achromobactor, Agrobacterium, Aérobacter, Alcaligènes, Azotobacter, Gluconobacter, Rhizobium, Sarcina, Pseudomonas, Flavobactérium, Brevibactérium ou Erwinia.

**3.** Procédé suivant la revendication 1, caractérisé en ce que la souche bactérienne productrice de cellulose appartient aux genres suivants: Acétobacter sp, Acétotacter aceti, Acetobacter aceti subsp-xylinum, Acetobacter acetigenum, Acetobacter pastorianus, Acetobacter hansenii, Acetobacter liquefaciens.

**4.** Procédé suivant la revendication 1 à 3, caractérisé en ce que la production de cellulose de la souche bactérienne est essentiellement exocellulaire.

**5.** Procédé suivant la revendication 1 à 4, caractérisé en ce que le procédé est un procédé discontinu ou semi-continu.

**6.** Procédé suivant la revendication 1 à 5, caractérisé en ce que durant la phase de production de la cellulose, l'aération est maintenue inférieure à 1,5 ppm de préférence à 0,5 ppm d'O2 dissous dans le réacteur.

**7.** Procédé suivant la revendication 1 à 6, caractérisé en ce que les moyens permettant la fixation de microorganismes sont des structures solides solidaires du réacteur.

**8.** Procédé suivant la revendication 1 à 7, caractérisé en ce que les moyens permettant la fixation de microorganismes sont des structures amovibles non solidaires du réacteur.

**9.** Procédé suivant l'une des revendications 1 à 8, caractérisé en ce que le milieu de production contient de 20 à 150 g/l d'hydrate de carbone.

**10.** Procédé suivant l'une des revendications 1 à 9, caractérisé en ce que les hydrates de carbone sont essentiellement des hexoses.

**11.** Procédé suivant l'une des revendications 1 à 10, caractérisé en ce que le milieu de production contient à titre d'hydrate de carbone:
  - du saccharose et/ou
  - des amidons ou leurs dérivés et analogues, y compris glucose.

**12.** Procédé selon l'une des revendication 1 à 11, caractérisé en ce que la concentration en hydrate de carbone est comprise entre 25 et 100 g/l.

**13.** Procédé suivant l'une quelconque des revendications 1 à 12, caractérisé en ce que le milieu de culture contient un sel de magnésium.

**14.** Procédé selon l'une quelconque des revendications 1 à 13, caractérisé en ce que le pH du milieu est inférieur à pH 5,5.

**15.** Procédé selon l'une quelconque des revendications 1 à 14, caractérisé en ce que le milieu de production contient de l'acide citrique.

**16.** Procédé selon l'une des revendications 1 à 15, caractérisé en ce que la biomasse est maintenue minimum en limitant l'apport d'azote et/ou de phosphore.

**17.** Procédé de l'une des revendications 1 à 16, caractérisé en ce que les conditions de production sont choisies de façon à conduire en fin de phase de production de la cellulose à une lyse ou autolyse des cellules.

**18.** Procédé selon l'une quelconque des revendications 1 à 17, caractérisé en ce que la cellulose est purifiée par traitement de la cellulose brute obtenue par de l'acide acétique.

**19.** Procédé selon la revendication 18, caractérisé en ce que la purification est effectuée par traitement à l'acide acétique à chaud, suivi par un lavage.

**20.** Procédé selon l'une des revendications 18 et 19, caractérisé en ce que la cellulose est lavée avec une base.

**Claims**

**1.** Process for producing cellulose of bacterial origin from material of plant origin containing carbohydrates, in a bioreactor in submerged culture, characterized in that in the production medium comprising said carbohydrates, at least one cellulose producing bacterial strain is cultivated and wherein said bioreactor comprises means enabling the fixation of the microorganisms and the development of the cellulose fibers and wherein during the phase of production of the cellulose, the aeration is low and stirring is maintained with minimum shear to facilitate the growth of the cellulose fibers.

**2.** Process according to claim 1, characterized in that the cellulose-producing bacterial strain belongs to the following genera: Achromobacter, Agrobacterium, Aerobacter, Alcaligenes, Azotobacter, Gluconobacter, Rhizobium, Sarcina, Pseudomonas, Flavobacterium, Brevibacterium or Erwinia.

**3.** Process according to claim 1, characterized in that the cellulose-producing bacterial strain belongs to the following genera: Acetobacter sp, Acetobacter aceti, Acetobacter aceti subsp-xylinum, Acetobacter acetigenum, Acetobacter pastorianus, Acetobacter hansenii, Acetobacter liquefaciens.

**4.** Process according to claim 1 to 3, characterized in that the cellulose production of the bacterial strain is essentially exocellular.

**5.** Process according to claim 1 to 4, characterized in that the process is a discontinuous or semi-continuous process.

**6.** Process according to claim 1 to 5, characterized in that during the production phase of the cellulose, the aeration is maintained less than 1.5 ppm preferably than 0.5 ppm of dissolved $O_2$ in the reactor.

**7.** Process according to claim 1 to 6, characterized in that the means enabling the fixation of microorganisms are solid structures immovably fixed to the reactor.

8. Process according to claim 1 to 7, characterized in that the means enabling the fixation of microorganisms are removable structures not immovably fixed to the reactor.

9. Process according to one of claims 1 to 8, characterized in that the production medium contains from 20 to 150 g/l of carbohydrate.

10. Process according to one of claims 1 to 9, characterized in that the carbohydrates are essentially hexoses.

11. Process according to one of claims 1 to 10, characterized in that the production medium contains as carbohydrate:
    - saccharose and/or
    - starches or their derivatives and analogs, including glucose.

12. Process according to one of claims 1 to 11, characterized in that the concentration of carbohydrate is comprised between 25 and 100 g/l.

13. Process according to any one of claims 1 to 12, characterized in that the culture medium contains a magnesium salt.

14. Process according to any one of claims 1 to 13, characterized in that the pH of the medium is less than pH 5.5.

15. Process according to any one of claims 1 to 14, characterized in that the production medium contains citric acid.

16. Process according to any one of claims 1 to 15, characterized in that the biomass is kept to the minimum by limiting the contribution of nitrogen and/or of phosphorus.

17. Process according to any one of claims 1 to 16, characterized in that the production conditions are selected so as to lead at the end of the production phase of the cellulose to a lysis or autolysis of the cells.

18. Process according to any one of claims 1 to 17, characterized in that the cellulose is purified by treatment of the crude cellulose obtained with acetic acid.

19. Process according to claim 18, characterized in that the purification is carried out by treatment with hot acetic acid, followed by washing.

20. Process according to anyone of claims 18 and 19, characterized in that the cellulose is washed with a base.

**Patentansprüche**

1. Verfahren zur Herstellung von Cellulose bakteriellen Ursprungs aus einem pflanzlichen Ausgangsmaterial, das Kohlenhydrate enthält, in einem Bioreaktor in Submers-Kultur, dadurch gekennzeichnet, daß man in dem Produktionsmedium, das die genannten Kohlenhydrate enthält, mindestens einen Cellulose bildenden Bakterienstamm kultiviert (züchtet), und daß der genannte Bioreaktor Einrichtungen aufweist, welche die Fixierung von Mikroorganismen und die Entwicklung von Cellulosefasern erlaubt und daß während der Phase der Bildung der Cellulose die Belüftung gering ist und mit einer minimalen Scherkraft gerührt wird, um das Wachsen der Cellulosefasern zu fördern.

2. Verfahren nach Anspruch 1, dadurch gekennzeichnet, daß der Cellulose bildende Bakterienstamm zu den folgenden Genus gehört: Achromobacter, Agrobacterium, Aerobacter, Alcaligenes, Azotobacter, Gluconobacter, Rhizobium, Sarcina, Pseudomonas, Flavobacterium, Brevibacterium oder Erwinia.

3. Verfahren nach Anspruch 1, dadurch gekennzeichnet, daß der Cellulose bildende Bakterienstamm zu den folgenden Genus gehört: Acetobacter sp., Acetobacter aceti, Acetobacter aceti subsp.-xylinum,

11

Acetobacter acetigenum, Acetobacter pastorianus, Acetobacter hansenii, Acetobacter liquefaciens.

4. Verfahren nach den Ansprüchen 1 bis 3, dadurch gekennzeichnet, daß die Bildung von Cellulose durch den Bakterienstamm im wesentlichen exocellulär erfolgt.

5. Verfahren nach den Ansprüchen 1 bis 4, dadurch gekennzeichnet, daß das Verfahren ein diskontinuierliches oder halbkontinuierliches Verfahren ist.

6. Verfahren nach den Ansprüchen 1 bis 5, dadurch gekennzeichnet, daß während der Phase der Bildung der Cellulose die Belüftung unterhalb 1,5 ppm, vorzugsweise unterhalb 0,5 ppm $O_2$, gelöst in dem Reaktor, gehalten wird.

7. Verfahren nach den Ansprüchen 1 bis 6, dadurch gekennzeichnet, daß die Einrichtungen, welche die Fixierung von Mikroorganismen erlauben, feste, mit dem Reaktor verbundene Strukturen sind.

8. Verfahren nach den Ansprüchen 1 bis 7, dadurch gekennzeichnet, daß die Einrichtungen, welche die Fixierung von Mikroorganismen erlauben, auswechselbare, mit dem Reaktor nicht verbundene Strukturen sind.

9. Verfahren nach einem der Ansprüche 1 bis 8, dadurch gekennzeichnet, daß das Produktionsmedium 20 bis 150 g Kohlenhydrat pro Liter enthält.

10. Verfahren nach einem der Ansprüche 1 bis 9, dadurch gekennzeichnet, daß die Kohlenhydrate im wesentlichen Hexosen sind.

11. Verfahren nach einem der Ansprüche 1 bis 10, dadurch gekennzeichnet, daß das Produktionsmedium als Kohlenhydrat enthält:
    - Saccharose und/oder
    - Stärken oder ihre Derivate und Analoga einschließlich Glucose.

12. Verfahren nach einem der Ansprüche 1 bis 11, dadurch gekennzeichnet, daß die Kohlenhydrat-Konzentration zwischen 25 und 100 g/l liegt.

13. Verfahren nach einem der Ansprüche 1 bis 12, dadurch gekennzeichnet, daß das Kulturmedium ein Magnesiumsalz enthält.

14. Verfahren nach einem der Ansprüche 1 bis 13, dadurch gekennzeichnet, daß der pH-Wert des Mediums unterhalb 5,5 liegt.

15. Verfahren nach einem der Ansprüche 1 bis 14, dadurch gekennzeichnet, daß das Produktionsmedium Citronensäure enthält.

16. Verfahren nach einem der Ansprüche 1 bis 15, dadurch gekennzeichnet, daß die Biomasse bei einem Minimum gehalten wird durch Begrenzung der Stickstoff- und/oder Phosphorzufuhr.

17. Verfahren nach einem der Ansprüche 1 bis 16, dadurch gekennzeichnet, daß die Produktionsbedingungen so gewählt werden, daß sie am Ende der Cellulosebildungsphase zu einer Lysis oder Autolysis der Zellen führen.

18. Verfahren nach einem der Ansprüche 1 bis 17, dadurch gekennzeichnet, daß die Cellulose durch Behandlung der erhaltenen rohen Cellulose mit Essigsäure gereinigt wird.

19. Verfahren nach Anspruch 18, dadurch gekennzeichnet, daß die Reinigung durch Behandlung mit warmer Essigsäure und anschließendes Waschen durchgeführt wird.

20. Verfahren nach einem der Ansprüche 18 und 19, dadurch gekennzeichnet, daß die Cellulose mit einer Base gewaschen wird.